(19) Europäisches Patentamt
European Patent Office
Office européen des brevets

(11) EP 2 934 698 B1

(12) **FASCICULE DE BREVET EUROPEEN**

(45) Date de publication et mention de la délivrance du brevet:
**30.01.2019 Bulletin 2019/05**

(21) Numéro de dépôt: **13826885.9**

(22) Date de dépôt: **20.12.2013**

(51) Int Cl.:
***A61Q 17/04*** (2006.01) ***A61K 8/29*** (2006.01)
***A61K 8/35*** (2006.01) ***A61K 8/37*** (2006.01)
***A61K 8/46*** (2006.01) ***A61K 8/58*** (2006.01)
***A61K 8/40*** (2006.01) ***A61K 8/34*** (2006.01)

(86) Numéro de dépôt international:
**PCT/IB2013/061219**

(87) Numéro de publication internationale:
**WO 2014/097260 (26.06.2014 Gazette 2014/26)**

# (54) COMPOSITION A BASE DE TRANS-RESVERATROL OU D'UN DERIVE DE TRANS-RESVERATROL

TRANSRESVERATROL- ODER TRANSRESVERATROLDERIVAT ZUSAMMENSETZUNG

TRANS-RESVERATROL OR TRANS-RESVERATROL DERIVATIVE COMPOSITION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.12.2012 FR 1262604**
**10.01.2013 US 201361751052 P**

(43) Date de publication de la demande:
**28.10.2015 Bulletin 2015/44**

(73) Titulaire: **L'Oréal**
**75008 Paris (FR)**

(72) Inventeurs:
- **CHEVALIER, Véronique**
**F-94440 Villecresnes (FR)**
- **THEROUIN-KOELY, Sandrine**
**94152 Chevilly-Larue (FR)**

(74) Mandataire: **Nony**
**11 rue Saint-Georges**
**75009 Paris (FR)**

(56) Documents cités:
**WO-A1-2012/069362 WO-A1-2012/069363**
**US-A1- 2012 213 845**

- **CRAIG A BONDA: "PHOTOSTABILIZATION OF RESVERATROL WITH ALKOXYCRYLENE COMPOUNDS", IP.COM JOURNAL, IP.COM INC., WEST HENRIETTA, NY, US, 6 avril 2011 (2011-04-06), XP013145736, ISSN: 1533-0001**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 934 698 B1

## Description

La présente invention se rapporte au domaine des compositions comprenant du *trans*-resvératrol et/ou au moins un dérivé du *trans*-resvératrol en particulier à titre d'agent antioxydant, anti-âge ou dépigmentant.

Le resvératrol est un polyphénol de la classe des stilbènes qui existe sous une forme *cis* et une forme *trans* représentées ci-dessous.

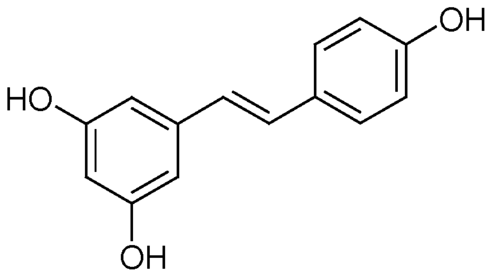

*trans*-resvératrol

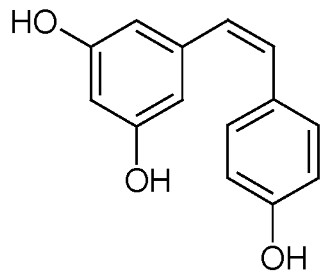

*cis*-resvératrol

Le *trans*-resvératrol est connu pour avoir de nombreuses propriétés biologiques telles qu'une activité antioxydante, anti-inflammatoire ou antitumorale.

Cependant, le *trans*-resvératrol est une molécule photosensible qui se transforme de manière irréversible en son isomère *cis* sous exposition à la lumière, or l'isomère trans est la forme la plus active.

Ainsi, il demeure un besoin de disposer d'une composition contenant du *trans*-resvératrol, stable notamment à la lumière.

Les inventeurs ont constaté qu'il était possible d'obtenir une telle composition, en introduisant des filtres UVA et UVB au sein de ladite composition.

Ainsi, selon un premier de ses aspects, la présente invention a pour objet une composition cosmétique ou dermatologique comprenant, dans un milieu physiologiquement acceptable, au moins du *trans*-resvératrol et/ou au moins un dérivé de *trans*-resvératrol choisi parmi les esters, les glucosides et les phosphates du *trans*-resvératrol, et un système filtrant organique comprenant au moins un filtre UVA et au moins un filtre UVB et/ou au moins un filtre mixte UVA-UVB,
ladite composition possédant un SPF supérieur ou égal à 15 et un PPD supérieur ou égal à 5, ledit filtre UVA étant un filtre hydrosoluble UVA.

De préférence, le *trans*-resvératrol ou dérivé de *trans*-resvératrol est présent dans une teneur allant de 0,001 à 10 % en poids par rapport au poids total de la composition.

En particulier, la composition selon l'invention, est caractérisée en ce que le rapport SPF/PPD va de 1 à 3, de préférence, de 1,3 à 2,7 et mieux de 1,5 à 2,5.

Selon un deuxième aspect, l'invention vise un procédé de stabilisation, en particulier de photostabilisation, d'une composition comprenant du *trans*-resvératrol et/ou au moins un dérivé de *trans*-resvératrol choisi parmi les esters, les glucosides et les phosphates du *trans*-resvératrol comprenant une étape lors de laquelle on ajoute à ladite composition un système filtrant organique comprenant au moins un filtre UVA et au moins un filtre UVB et/ou au moins un filtre mixte UVA-UVB, ledit filtre UVA étant un filtre hydrosoluble UVA, en une quantité telle que le SPF de la composition est porté à une valeur supérieure ou égale à 15 et le PPD de la composition est porté à une valeur supérieure ou égale 5.

L'invention vise aussi l'utilisation d'un système filtrant comprenant au moins un filtre UVA et au moins un filtre UVB et/ou au moins un filtre mixte UVA-UVB, ledit filtre UVA étant un filtre hydrosoluble UVA, pour stabiliser notamment photostabiliser une composition comprenant du *trans*-resvératrol et/ou au moins un dérivé de *trans*-resvératrol choisi parmi les esters, les glucosides et les phosphates du *trans*-resvératrol.

Cette utilisation comprenant en particulier l'ajout à ladite composition dudit système filtrant organique en une quantité telle que le SPF de la composition est porté à une valeur supérieure ou égale à 15 et le PPD de la composition est porté à une valeur supérieure ou égale 5.

De manière surprenante, les inventeurs ont constaté que le *trans*-resvératrol présent dans la composition selon l'invention était particulièrement stable après avoir été exposé à la lumière.

Par « dérivé du *trans*-resvératrol » au sens de la présente invention, on entend les esters, glucosides et les phosphates du *trans*-resvératrol.

Ces composés possèdent tous les mêmes propriétés biologiques que le *trans*-resvératrol et sont de stéréochimie *trans.* Tout comme le *trans*-resvératrol, ils sont photosensibles, et se transforment irréversiblement en leur isomère *cis* sous exposition à la lumière.

Dans la suite de la description par « *trans*-resvératrol » on entend le *trans*-resvératrol ou au moins un dérivé de *trans*-resvératrol ou un mélange de *trans*-resvératrol et de dérivé(s) de *trans*-resvératrol.

Par composition « stable » au sens de la présente invention on entend une composition dans laquelle le *trans*-resvératrol se dégrade peu en particulier sous exposition à la lumière, notamment par transformation en son isomère *cis.* De préférence, une composition stable selon l'invention comprend au moins 70 % en poids de resvératrol, avantageusement au moins 75 % en poids de resvératrol, en particulier au moins 80 % en poids de resvératrol par rapport au poids total de resvératrol initialement introduit dans la composition.

Par « SPF » (Sun Protection Factor) au sens de l'invention on entend le facteur de protection solaire, qui mesure le niveau de protection contre les UVB. La valeur du SPF correspond au rapport entre la durée minimale qu'il faut pour obtenir un coup de soleil avec une composition antisolaire et celle sans produit.

Il s'exprime mathématiquement par le rapport de la dose de rayonnement UV nécessaire pour atteindre le seuil érythématogène avec le filtre UV sur la dose de rayonnement UV nécessaire pour atteindre le seuil érythématogène sans filtre UV. Ce facteur concerne donc l'efficacité de la protection dont le spectre d'action biologique est centré dans l'UVB et par conséquent, rend compte de la protection vis à vis de ce rayonnement UV-B.

Pour caractériser la protection vis à vis des UV-A, la méthode PPD (Persistent Pigment Darkening), qui mesure la couleur de la peau observée 2 à 4 heures après une exposition de la peau aux UV-A, est particulièrement recommandée et utilisée. Cette méthode est adoptée depuis 1996 par la Japanese Cosmetic Industry Association (JCIA) en tant que procédure officielle de test pour l'étiquetage UV-A des produits et est fréquemment utilisée par les laboratoires de tests en Europe et aux Etats-Unis; (Japan Cosmetic Industry Association Technical Bulletin. Measurement Standards for UVA protection efficacy. Issued November 21, 1995 and effective of January 1, 1996).

Le facteur de protection solaire UVA PPD (FP UVAppd) s'exprime mathématiquement par le rapport de la dose de rayonnement UV-A nécessaire pour atteindre le seuil de pigmentation avec le filtre UV (MPPDp) sur la dose de rayonnement UV-A nécessaire pour atteindre le seuil de pigmentation sans filtre UV (MPPDnp).

$$\text{FP UVA}_{\text{PPD}} = \frac{\text{MPPDp}}{\text{MPPDnp}}$$

La présente demande décrit également l'utilisation cosmétique d'une quantité efficace de *trans*-resvératrol ou d'au moins un dérivé de *trans*-resvératrol tel que défini précédemment, pour lutter contre les signes cutanés du vieillissement, notamment induits par un stress oxydatif.

En effet, le stress oxydatif est notamment lié à des agents extérieurs comme les rayonnements ultraviolets, la pollution, un stress thermique ou encore diverses toxines telles que des agents chimiques avec lesquels la peau peut être en contact (tabac, métaux lourds, polluants atmosphériques, etc).

En particulier, l'utilisation vise à atténuer les signes cutanés du vieillissement, en particulier les signes du vieillissement cutané d'origine actinique tel que le photo-vieillissement.

Parmi les signes cutanés du vieillissement, notamment induits par un stress oxydatif, en particulier d'origine actinique, on cite notamment une perte de fermeté et/ou d'élasticité et/ou de tonicité et/ou de souplesse de la peau, la formation des rides et des ridules, les rides d'expression, en particulier au niveau du front et de l'espace intersourcillier, les rides et/ou ridules péri-buccales, et/ou le relâchement au niveau du contour des lèvres, en particulier au niveau de la lèvre blanche (zone située entre la lèvre supérieure et le nez), un aspect terne du teint, l'aspect papyracé de la peau.

Cette utilisation sera notamment destinée aux personnes à peau mature, voire très mature.

Par « peaux matures » selon l'invention, on entend notamment des personnes ayant au moins 40 ans.

Par « peaux très matures » selon l'invention, on entend notamment des personnes ayant au moins 50 ans, en particulier au moins 60 ans, voire 65 ans.

Les rides d'expression sont produites sous l'effet de la contrainte exercée sur la peau par les muscles peauciers qui permettent les mimiques. Selon la forme du visage, la fréquence des mimiques et les tics éventuels, elles peuvent apparaître dès l'enfance. Certains facteurs environnementaux tels que le stress oxydatif, l'exposition au soleil, de même que l'âge n'interviennent pas dans leur genèse mais peuvent les creuser davantage et les rendre permanentes. Les rides d'expression se caractérisent par la présence de sillons sur le pourtour des orifices que constituent le nez (sillons nasogéniens), la bouche (rides para-buccales et rides dites de l'amertume) et les yeux (rides de la patte d'oie), autour desquels se situent les muscles peauciers, ainsi qu'entre les sourcils (rides de la glabelle ou du lion) et sur le front. En particulier, on cherchera à prévenir et/ou lisser les rides du front et de l'espace intersourcillier.

L'aspect papyracé de la peau se caractérise par une modification de l'aspect visuel, ainsi que de la tenue au toucher, de la peau. Plus précisément, la peau revêt visuellement l'aspect d'un papier à cigarette lui donnant une apparence similaire à celle d'une feuille de papyrus. En outre, lorsqu'elle est légèrement pincée entre le pouce et l'index, la peau forme des plis fins, aigus et nombreux ayant l'apparence d'un papier froissé. Enfin, le toucher de la peau montre que ses parties superficielles sont comme flottantes sur les parties profondes, donnant à la peau, au stade très évolué

d'aspect papyracé, l'apparence d'un papier chiffonné. L'aspect papyracé de la peau est visible sur le visage et encore plus caractéristique sur le dos des mains de personnes âgées.

Les compositions conformes à l'invention destinées à prévenir et/ou lisser les rides d'expression seront appliquées sur le visage.

Les compositions conformes à l'invention destinées à prévenir et/ou traiter l'aspect papyracé de la peau seront notamment appliquées sur le dos des mains.

Les compositions conformes à l'invention destinées à diminuer l'apparence et/ou la visibilité des pores seront appliquées en particulier au niveau de la zone T (front, nez, joues, menton).

L'utilisation d'actifs antioxydants peut s'avérer utile dans des compositions anti-âge ou encore dans des compositions dépigmentantes.

L'utilisation selon l'invention peut également être plus particulièrement destinée aux personnes à peau grasse.

Les compositions conformes à l'invention peuvent être des produits de maquillage ou de soin des matières kératiniques, en particulier de la peau.

Plus précisément les produits de maquillage peuvent être de type fonds de teint, fards à joues ou à paupières, produits anti-cerne, blush, ou encore un produit de maquillage du corps ou de coloration de la peau.

Les produits de soin de la peau peuvent être une composition de protection, de traitement ou de soin pour le visage, pour les mains, pour les pieds, pour les grands plis anatomiques ou pour le corps par exemple crèmes de jour, crème de nuit, composition antisolaire, laits corporels de protection ou de soin, laits après-solaire, lotion, gel ou mousse pour le soin de la peau, composition de bronzage artificiel; ou une composition après-rasage.

La présente demande décrit également un procédé de traitement cosmétique de la peau et/ou de ses phanères, destiné à prévenir et/ou traiter le vieillissement cutané, notamment induit par un stress oxydatif comprenant au moins une étape consistant à appliquer sur une peau et/ou ses phanères présentant des signes de vieillissement cutané induit par un stress oxydatif au moins une quantité efficace d'au moins une composition selon l'invention.

La présente demande décrit encore sur un procédé de traitement cosmétique de la peau et/ou de ses phanères, visant à protéger la peau et/ou ses phanères des effets du stress oxydatif, en particulier des effets des rayonnements UV, et/ou des toxines et/ou de la pollution, comprenant au moins une étape consistant à appliquer sur la peau et/ou ses phanères au moins une quantité efficace d'au moins une composition selon l'invention. La composition selon l'invention est adaptée à une application topique.

Par « peau », on entend la peau du visage et/ou du corps, le cuir chevelu et les semi-muqueuses (lèvres). De préférence, il s'agira de la peau du visage et/ou du corps et/ou des lèvres.

Par « phanères », on entend les cheveux, les poils, les cils, les ongles, et de préférence les cheveux.

La présente demande décrit enfin un procédé de traitement cosmétique destiné à prévenir l'irritation cutanée induite par un stress oxydatif comprenant au moins une étape consistant à appliquer sur une peau susceptible de subir un stress oxydatif au moins une quantité efficace d'au moins une composition telle que définie précédemment.

Le terme « stress oxydatif » tel qu'employé dans la présente demande recouvre l'ensemble des dommages causés chez un sujet par des radicaux libres.

L'ampleur des dommages engendrés par ce stress oxydatif dépend de la rapidité avec laquelle les radicaux libres sont créés et ensuite inactivés par des antioxydants.

## ***TRANS*-RESVERATROL**

Le resvératrol existe à l'état naturel sous sa forme *cis* et *trans,* mais également sous d'autres formes comme par exemple une forme glucosylée. Il est présent dans de nombreuses plantes et fruits. On le trouve notamment dans la renouée du Japon (*Fallopia japonica* également connu sous le nom de *Polygonum cupistadum* ou encore *Reynoutria japonica*) ou dans les raisins comme par exemples ceux issus de l'espèce de vigne *Vitis vinifera.* Plus particulièrement on trouve par exemple le resvératrol dans les mûres, le vin ou les cacahuètes.

De manière générale, on trouve du resvératrol dans les familles de plantes suivantes : *Vitaceae, Myrtaceae, Dipterocarpaceae, Cyperaceae, Gnetaceae, Fabaceae, Pinaceae, Polygonaceae, Moraceae, Fagaceae, Liliaceae...*

Le *trans*-resvératrol présent dans la composition selon l'invention peut se trouver sous la forme d'un extrait de plante contenant du *trans*-resvératrol.

Selon un mode de réalisation particulier de l'invention, ledit *trans*-resvératrol et/ou au moins un dérivé de *trans*-resvératrol est présent dans la composition selon l'invention dans une teneur allant de 0,01 à 5 % en poids, de préférence de 0,05 à 2 % en poids par rapport au poids total de la composition.

Au sens de l'invention, par teneur on entend teneur en matière active.

La présente invention vise encore un procédé de préparation de ladite composition.

## FILTRES ORGANIQUES

Comme précisé précédemment, une composition selon l'invention comprend un système filtrant organique comprenant au moins un filtre UVA et au moins un filtre UVB et ou au moins un filtre mixte UVA-UVB, ledit filtre UVA étant un filtre hydrosoluble UVA.

### *Filtres UVA*

Le ou les filtres UVA selon la présente invention peu(ven)t être choisi(s) parmi les filtres suivants.

### Filtres hydrosolubles UVA

- Les dérivés du camphre tel que le Terephtalylidène Dicamphor Sulfonic Acid fabriqué sous le nom « MEXORYL SX » par CHIMEX,
- Les dérivés bis-benzoazolyle tels que décrits dans les brevets EP 669 323, et US 2,463,264 et plus particulièrement le composé Disodium Phenyl Dibenzimidazo tetra-sulfonate vendu sous le nom commercial « NEO HELIOPAN AP » par SYMRISE ;

Selon un mode de réalisation préféré ledit au moins un filtre UVA est choisi parmi les dérivés hydrosolubles du camphre.

Plus particulièrement, ledit au moins un filtre UVA est choisi parmi le téréphtalylidène dicamphor sulfonic acid. De préférence, une composition selon l'invention est caractérisée en ce que ledit au moins un filtre UVA est présent dans une teneur allant de 1 à 20 %, en particulier, de 2 à 15 % en poids par rapport au poids total de la composition.

Une composition selon l'invention peut comprendre en outre au moins un filtre UVA hydrophobe.

Des exemples de filtres UVA hydrophobes sont décrits ci-dessous:

Dérivés du dibenzoylméthane :

- Butyl Methoxydibenzoylméthane vendu notamment sous le nom commercial « PARSOL 1789 » par DSM Nutritional Products, Inc ;
- Isopropyl Dibenzoylméthane ;

Aminobenzophénones :

- 2-(4-diéthylamino-2-hydroxybenzoyl)-benzoate de n-hexyle vendu notamment sous le nom commercial « UVINUL A +» par BASF ;

Dérivés anthraniliques :

- Menthyl anthranilate vendu notamment sous le nom commercial « NEO HELIOPAN MA » par SYMRISE ;

Dérivés de 4,4-diarylbutadiène :

- 1,1-dicarboxy (2,2'-diméthyl-propyl)-4,4-diphénylbutadiène ;

Dérivés de mérocyanine :

- Octyl-5-N,N-diéthylamino-2-phénysulfonyl-2,4-pentadiénoate ;

En particulier, une composition selon l'invention comprend en outre au moins un filtre UVA choisi parmi les dérivés hydrophobes du dibenzoylméthane, de préférence le butyl méthoxydibenzoyleméthane.

***Filtres UVB***

Le ou les filtres UVB selon la présente invention peu(ven)t être choisi(s) parmi les filtres suivants.

**1) Filtres hydrophobes UVB**

Para-aminobenzoates :

- Ethyl PABA ;
- Ethyl Dihydroxypropyl PABA ;
- Ethylhexyl Diméthyl PABA (ESCALOL 507 de ISP) ;

Dérivés salicyliques :

- Homosalate vendu notamment sous le nom « Eusolex HMS » par Rona/EM Industries ;
- Ethylhexyl Salicylate vendu notamment sous le nom « NEO HELIOPAN OS » par SYMRISE ;
- Dipropylèneglycol Salicylate vendu notamment sous le nom « DIPSAL » par SCHER;
- TEA Salicylate et sous le nom « NEO HELIOPAN TS » par SYMRISE ;

Cinnamates

- Ethylhexyl Méthoxycinnamate vendu notamment sous le nom commercial « PARSOL MCX » par DSM Nutritional Products, Inc. ;
- Isopropyl Méthoxy cinnamate ;
- Isoamyl Méthoxy cinnamate vendu notamment sous le nom commercial « NEO HELIOPAN E 1000 » par SYMRISE ;
- Diisopropyl Méthylcinnamate ;
- Cinnoxate ;
- Glycéryl Ethylhexanoate Diméthoxycinnamate ;

Dérivés de β,β'-diphénylacrylate :

- Octocrylène, vendu notamment sous le nom commercial « UVINUL N539 » par BASF ;
- Etocrylène, vendu notamment sous le nom commercial « UVINUL N35 » par BASF;

Dérivés du benzylidène camphre :

- 3-Benzylidène camphre fabriqué sous le nom « MEXORYL SD » par CHIMEX ;
- Méthylbenzylidène camphre vendu notamment sous le nom « EUSOLEX 6300 » par MERCK ;
- Polyacrylamidométhyle Benzylidène Camphre fabriqué sous le nom « MEXORYL SW » par CHIMEX ;

Dérivés de triazine :

- Ethylhexyl triazone vendu notamment sous le nom commercial « UVINUL T150 » par BASF ;
- Diéthylhexyl Butamido Triazone vendu notamment sous le nom commercial « UVASORB HEB » par SIGMA 3V ;
- 2,4,6-tris(4'-amino benzalmalonate de dinéopentyle)-s-triazine ;
- 2,4,6-tris(4'-amino benzalmalonate de diisobutyle)-s- triazine ;
- 2,4-bis(4'-amino benzalmalonate de dinéopentyle)-6-(4'-aminobenzoate de n-butyle)-s-triazine ;
- 2,4-bis(4'-amino benzoate de n-butyle)-6-(aminopropyltrisiloxane)-s-triazine ;
- les filtres triazines symétriques décrits dans le brevet US 6,225,467, la demande WO 2004/085412 (voir composés 6 et 9) ou le document « Symetrical Triazine Derivatives » IP.COM Journal, IP.COM INC WEST HENRIETTA, NY, US (20 septembre 2004) notamment les 2,4,6-tris-(biphényl)-1,3,5-triazine (en particulier la 2,4,6-tris(biphényl-4-yl-1,3,5-triazine) et la 2,4,6-tris(terphenyl)-1,3,5-triazine, ces deux derniers filtres étant décrits dans les demandes de BEIERSDORF WO 06/035000, WO 06/034982, WO 06/034991, WO 06/035007, WO 2006/034992, WO 2006/034985).

Dérivés d'imidazolines :

- Ethylhexyl Diméthoxybenzylidene Dioxoimidazoline Propionate,

Dérivés du benzalmalonate :

- Polyorganosiloxanes à fonction benzalmalonate tels que le Polysilicone-15 vendu notamment sous la dénomination commerciale « PARSOL SLX » par DSM Nutritional Products, Inc. ;
- Di-neopentyl 4'-méthoxybenzalmalonate ;

**2) Filtres hydrosolubles UVB**

Les dérivés d'acide p-aminobenzoïque (PABA) suivants :

- PABA,
- Glyceryl PABA et
- PEG-25 PABA vendu notamment sous la dénomination commerciale « UVINUL P25 » par BASF.
- le Phenylbenzimidzaole Sulfonic Acid vendu notamment sous la dénomination commerciale « EUSOLEX 232 » par MERCK,
- l'acide férulique,
- l'acide salicylique,
- le DEA methoxycinnamate,
- le benzylidène camphre Acide Sulfonique fabriqué sous le nom « MEXORYL SL » par CHIMEX,
- le camphre Benzalkonium Methosulfate fabriqué sous le nom « MEXORYL SO » par CHIMEX.

Selon un mode de réalisation préféré, le au moins un filtre UVB compris dans une composition selon l'invention est un filtre hydrophobe UVB.

En particulier, ledit au moins un filtre hydrophobe UVB est choisi parmi les dérivés salicyliques, les cinnamates, les dérivés de β,β'-diphénylacrylate, les dérivés de triazine et leurs mélanges.

De préférence, le moins un filtre UVB est choisi parmi l'éthylhexyl salicylate, l'éthylhexyl méthoxycinnamate, l'octocrylène, l'éthylhexyl triazone et leurs mélanges.

De préférence, une composition selon l'invention est caractérisée en ce que ledit au moins un filtre UVB est présent dans une teneur allant de 1 à 20 %, en particulier de de 5 à 15 %, et de préférence de 7 à 10 % en poids par rapport au poids total de la composition.

Selon une variante particulière de l'invention, ledit système filtrant organique utilisé dans l'invention contient voire est constitué d'un ou plusieurs filtre(s) mixte(s) UVA-UVB.

Avantageusement, ledit système filtrant organique utilisé dans l'invention contient un ou plusieurs filtres UVA, un ou plusieurs filtres UVB et un ou plusieurs filtres mixte UVA-UVB.

***Filtres mixtes UVA et UVB***

Ledit au moins un filtre mixte UVA-UVB peut être choisi parmi les filtres suivants.

**1) Filtres hydrophobes mixtes UVA et UVB**

Dérivés de benzophénone

- Benzophénone-1 vendu notamment sous le nom commercial « UVINUL 400 » par BASF ;
- Benzophénone-2 vendu notamment sous le nom commercial « UVINUL D50 » par BASF ;
- Benzophénone-3 ou Oxybenzone vendu notamment sous le nom commercial « UVINUL M40 » par BASF ;
- Benzophénone-6 vendu notamment sous le nom commercial « Helisorb 11 » par Norquay ;
- Benzophénone-8 vendu notamment sous le nom commercial « Spectra-Sorb UV-24 » par American Cyanamid ;
- Benzophénone-10 ;
- Benzophénone-11 ;
- Benzophénone-12 ;

Dérivés du phényl benzotriazole :

- Drométrizole Trisiloxane vendu notamment sous le nom « Silatrizole » par RHODIA CHIMIE ou fabriqué sous le nom « Mexoryl XL » par la société CHIMEX ;
- Méthylène bis-Benzotriazolyl Tétramethylbutylphénol, vendu sous forme solide notamment sous le nom commercial « MIXXIM BB/100 » par FAIRMOUNT CHEMICAL ou sous forme micronisée en dispersion aqueuse notamment sous le nom commercial « TINOSORB M » par CIBA SPECIALTY CHEMICALS ;

Dérivés bis-résorcinyl triazines

- Bis-Ethylhexyloxyphénol Méthoxyphenyl Triazine vendu notamment sous le nom commercial « TINOSORB S » par CIBA GEIGY ;

Dérivés de benzoxazole :

- 2,4-bis-[5-1(diméthylpropyl)benzoxazol-2-yl-(4-phenyl)-imino]-6-(2-ethylhexyl)-imino-1,3,5-triazine vendu notamment sous le nom d'Uvasorb K2A par Sigma 3V.

**2) Filtres hydrosolubles mixtes UVA et UVB**

Dérivés de benzophénone comportant au moins un radical sulfonique comme

- Benzophénone-4 vendu notamment sous le nom commercial « UVINUL MS 40 » par BASF,
- Benzophénone-5, et
- Benzophénone-9.

De préférence, ledit au moins un filtre mixte UVA-UVB selon l'invention est un filtre hydrophobe.

En particulier, ledit filtre mixte UVA-UVB est choisi parmi les dérivés du phényl benzotriazole et les dérivés bis-résorcinyl triazines, de manière préférée il est choisi parmi le drometrizole trisiloxane, le bis-ethylhexyloxyphénol méthoxyphenyl triazine, et leurs mélanges.

De préférence, une composition selon l'invention est caractérisée en ce que ledit au moins un filtre mixte UVA-UVB est présent dans une teneur allant de 1 à 20 %, en particulier de de 1 à 10 %, et de préférence de 1 à 4 % en poids par rapport au poids total de la composition.

Selon un mode de réalisation particulier, une composition de l'invention est caractérisée en ce que ledit système filtrant organique comprend au moins un filtre choisi parmi l'acide téréphtalylidène dicamphre sulfonique, le drometrizole trisiloxane et leurs mélanges.

Selon une première variante préférée, la composition selon l'invention comprend de l'octocrylène, de l'éthylhexyltriazone (Uvinul T150), du Drométrizole Trisiloxane (Mexoryl XL), du Butyl Methoxydibenzoylméthane (Avobenzone), du Terephtalylidène Dicamphor Sulfonic Acid (Mexoryl SX) avec ou sans $TiO_2$.

Selon une deuxième variante préférée, la composition selon l'invention comprend du Drométrizole Trisiloxane (Mexoryl XL), de l'éthylhexyl Méthoxycinnamate, du Terephtalylidène Dicamphor Sulfonic Acid (Mexoryl SX) et du $TiO_2$ traité, par exemple avec du stéarate d'aluminium.

Selon une troisième variante préférée, la composition selon l'invention comprend du Drométrizole Trisiloxane (Mexoryl XL), de l'Homosalate, de l'éthylhexyltriazone (Uvinul T150), de l'éthylhexyl Salicylate, de la Bis-Ethylhexyloxyphénol Méthoxyphenyl Triazine, du Butyl Methoxydibenzoylméthane (Avobenzone), et du Terephtalylidène Dicamphor Sulfonic Acid (Mexoryl SX).

Avantageusement, une composition selon l'invention, et plus particulièrement lorsqu'elle est sous la forme d'une émulsion, est caractérisée en ce que ledit système filtrant organique comprend au moins un filtre hydrosoluble et au moins un filtre hydrophobe, de préférence ledit filtre hydrosoluble est un filtre UVA.

Ainsi, le système filtrant organique comprend de préférence au moins un filtre hydrosoluble UVA et au moins un filtre hydrophobe UVB, en particulier, il consiste en l'association d'au moins un filtre hydrosoluble UVA et au moins un filtre hydrophobe UVB.

Plus particulièrement, la composition selon l'invention comprend les mélanges de filtres UV suivants en poids par rapport au poids total de la composition :

- de 1 à 5 % de Butyl Methoxydibenzoylméthane, de 0.1 à 3% d'éthylhexyltriazone, de 0.5 à 4 % de Terephtalylidène Dicamphor Sulfonic Acid , de 5 à 10% d'octocrylène, de 0.5 à 4 % de Drométrizole Trisiloxane et de 0.1 à 3% de $TiO_2$ par rapport au poids total de la composition ;
- de 1 à 5 % de Butyl Methoxydibenzoylméthane, de 3 à 7 % d'éthylhexyl Salicylate, de 0,1 à 3 % d'éthylhexyltriazone, de 1,5 à 6 % de Terephtalylidène Dicamphor Sulfonic Acid, de 1 à 5 % d'octocrylène, de 0,1 à 1 % de Drométrizole Trisiloxane, de 0,1 à 3 % de Bis-Ethylhexyloxyphénol Méthoxyphenyl Triazine et de 1 à 3 % de $TiO_2$ par rapport au poids total de la composition ;
- de 5 à 10 %, d'éthylhexyl Méthoxycinnamate, de 10 à 15 % de Terephtalylidène Dicamphor Sulfonic Acid, de 0.5 à 4 % de Drométrizole Trisiloxane et de 0,1 à 3 % de $TiO_2$ par rapport au poids total de la composition ;
- de 2 à 6 % de Butyl Methoxydibenzoylméthane, de 3 à 7% d'éthylhexyl Salicylate, de 0,1 à 3 % d'éthylhexyltriazone, de 1 à 5 % de Terephtalylidène Dicamphor Sulfonic Acid, de 1 à 5 % d'octocrylène, de 0.1 à 2 % de Drométrizole Trisiloxane, de 1 à 5 % de Bis-Ethylhexyloxyphénol Méthoxyphenyl Triazine et de 3 à 7% de $TiO_2$ par rapport au poids total de la composition ;
- de 0,1 à 2 % de Drométrizole Trisiloxane, de 4 à 8% d'Homosalate, de 1 à 5 % de Bis-Ethylhexyloxyphénol Méthoxyphenyl Triazine, de 2 à 6 % de Butyl Methoxydibenzoylméthane, de 1 à 5 % de Terephtalylidène Dicamphor Sulfonic Acid, de 3 à 7 % d'éthylhexyl Salicylate, de 1 à 5% d'éthylhexyltriazone.

## ADDITIFS

La composition selon l'invention peut en outre comporter des additifs comme par exemples des filtres inorganiques, des actifs, des charges, des corps gras, des polymères, des silicones.

### *Filtres inorganiques*

Les filtres inorganiques sont choisis parmi des pigments d'oxydes métalliques enrobés ou non dont la taille moyenne des particules primaires est préférentiellement comprise entre 5 nm et 100 nm (de préférence entre 10 nm et 50 nm) comme par exemple des pigments d'oxyde de titane (amorphe ou cristallisé sous forme rutile et/ou anatase), de fer, de zinc, de zirconium ou de cérium qui sont tous des agents photoprotecteurs UV bien connus en soi.

Les pigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

De façon connue, les silicones sont des polymères ou oligomères organo-siliciés à structure linéaire ou cyclique,

ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium.

Le terme "silicones" englobe également les silanes nécessaires à leur préparation, en particulier, les alkyl silanes.

Les silicones utilisées pour l'enrobage des pigments convenant à la présente invention sont de préférence choisies dans le groupe contenant les alkyl silanes, les polydialkylsiloxanes, et les polyalkylhydrogénosiloxanes. Plus préférentiellement encore, les silicones sont choisies dans le groupe contenant l'octyl triméthyl silane, les polydiméthylsiloxanes et les polyméthylhydro-génosiloxanes.

Bien entendu, les pigments d'oxydes métalliques avant leur traitement par des silicones, peuvent avoir été traités par d'autres agents de surface, en particulier par de l'oxyde de cérium, de l'alumine, de la silice, des composés de l'aluminium, des composés du silicium, ou leurs mélanges.

Les pigments enrobés sont plus particulièrement des oxydes de titane enrobés :

- de silice tels que le produit "SUNVEIL" de la société IKEDA et le produit "Eusolex T-AVO" de la société MERCK
- de silice et d'oxyde de fer tels que le produit "SUNVEIL F" de la société IKEDA,
- de silice et d'alumine tels que les produits "MICROTITANIUM DIOXIDE MT 500 SA" et "MICROTITANIUM DIOXIDE MT 100 SA" de la société TAYCA, "TIOVEIL" de la société TIOXIDE, et « Mirasun TiW 60 » de la société Rhodia,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tels que le produit "MICROTITANIUM DIOXIDE MT 100 TV, MT 100 TX, MT 100 Z, MT-01 de la société TAYCA, les produits "Solaveil CT-10 W", "Solaveil CT 100" et "Solaveil CT 200" de la société UNIQEMA,
- de silice, d'alumine et d'acide alginique tel que le produit "MT-100 AQ" de la société TAYCA,
- d'alumine et de laurate d'aluminium tel que le produit "MICROTITANIUM DIOXIDE MT 100 S" de la société TAYCA,
- d'oxyde de fer et de stéarate de fer tels que le produit "MICROTITANIUM DIOXIDE MT 100 F" de la société TAYCA,
- d'oxyde de zinc et de stéarate de zinc tels que le produit "BR351" de la société TAYCA,
- de silice et d'alumine et traités par une silicone tels que les produits "MICROTITANIUM DIOXIDE MT 600 SAS", "MICROTITANIUM DIOXIDE MT 500 SAS" ou "MICROTITANIUM DIOXIDE MT 100 SAS" de la société TAYCA,
- de silice, d'alumine, de stéarate d'aluminium et traités par une silicone tels que le produit "STT-30-DS" de la société TITAN KOGYO,
- de silice et traité par une silicone tel que le produit "UV-TITAN X 195" de la société KEMIRA, ou le produit SMT-100 WRS de la société TAYCA.
- d'alumine et traités par une silicone tels que les produits "TIPAQUE TTO-55 (S)" de la société ISHIHARA, ou "UV TITAN M 262" de la société KEMIRA,
  de triéthanolamine tels que le produit "STT-65-S" de la société TITAN KOGYO,
- d'acide stéarique tels que le produit "TIPAQUE TTO-55 (C)" de la société ISHIHARA,
- d'hexamétaphosphate de sodium tels que le produit "MICROTITANIUM DIOXIDE MT 150 W" de la société TAYCA.

D'autres pigments d'oxyde de titane traités avec une silicone sont de préférence le $TiO_2$ traité par l'octyl triméthyl silane et dont la taille moyenne des particules élémentaires est comprise entre 25 et 40 nm tel que celui vendu sous la dénomination commerciale "T 805" par la société DEGUSSA SILICES, le TiO2 traité par un polydiméthylsiloxane et dont la taille moyenne des particules élémentaires est de 21 nm tel que celui vendu sous la dénomination commerciale "70250 Cardre UF TiO2SI3" par la société CARDRE, le TiO2 anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.

Les pigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les pigments d'oxyde de zinc non enrobés, sont par exemple

- ceux commercialisés sous la dénomination "Z-cote" par la société Sunsmart ;
- ceux commercialisés sous la dénomination "Nanox" par la société Elementis ;
- ceux commercialisés sous la dénomination "Nanogard WCD 2025" par la société Nanophase Technologies ;

Les pigments d'oxyde de zinc enrobés sont par exemple

- ceux commercialisés sous la dénomination « Z-COTE HP1 » par la société SUNSMART (ZnO enrobé dimethicone) ;
- ceux commercialisés sous la dénomination "Oxide zinc CS-5" par la société Toshibi (ZnO enrobé par polymethylhydrogenesiloxane) ;
- ceux commercialisés sous la dénomination "Nanogard Zinc Oxide FN" par la société Nanophase Technologies (en dispersion à 40% dans le Finsolv TN, benzoate d'alcools en C12-C15) ;
- ceux commercialisés sous la dénomination "DAITOPERSION ZN-30" et "DAITOPERSION Zn-50" par la société Daito (dispersions dans cyclopolyméthylsiloxane/polydiméthylsiloxane oxyéthyléné, contenant 30 % ou 50 % de nano-oxydes de zinc enrobés par la silice et le polyméthylhydrogènesiloxane) ;
- ceux commercialisés sous la dénomination "NFD Ultrafine ZnO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "Escalol Z100" par la société ISP (ZnO traité alumine et dispersé dans le mélange methoxycinnamate d'ethylhexyle / copolymère PVP-hexadecene / methicone) ;
- ceux commercialisés sous la dénomination "Fuji ZnO-SMS-10" par la société Fuji Pigment (ZnO enrobé silice et polymethylsilsesquioxane) ;
- ceux commercialisés sous la dénomination "Nanox Gel TN" par la société Elementis (ZnO dispersé à 55% dans du benzoate d'alcools en C12-C15 avec polycondensat d'acide hydroxystéarique).

Les pigments d'oxyde de cérium non enrobé sont vendus par exemple sous la dénomination "COLLOIDAL CERIUM OXIDE" par la société RHONE POULENC.

Les pigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les pigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

***Particules composites***

La composition selon l'invention peut également comprendre, à titre de filtres inorganiques, des particules composites.

Les particules composites utilisables selon la présente invention comprennent une matrice et un filtre UV inorganique. La matrice comprend un ou plusieurs matériaux organiques et/ou inorganiques. Le filtre UV inorganique est généralement choisi parmi les oxydes métalliques.

Selon une variante préférée, la matrice est essentiellement constituée du matériau organique et/ou inorganique.

Les matériaux inorganiques utilisables dans la matrice selon la présente invention sont choisis dans le groupe formé par le mica, le mica synthétique, le talc, la sérécite, le nitrure de bore, le verre, le carbonate de calcium, le sulfate de baryum, l'hydroxyapatite, la silice, le silicate, le sulfate de magnésium, le carbonate de magnésium, le trisilicate de magnésium, l'oxyde d'aluminium, le silicate d'aluminium, le silicate de calcium, le phosphate de calcium, l'oxyde de magnésium, l'oxychlorure de bismuth, le kaolin, l'hydrotalcite, les argiles minérales, les argiles synthétiques et leurs mélanges.

Les matériaux organiques utilisables pour former la matrice sont choisis dans le groupe formé par les poly(méth)acrylates, polyamides, silicones, polyuréthanes, polyéthylènes, polypropylènes, polystyrènes, polyhydroxyalkanoates, polycaprolactames, poly(butylène)succinates, polysaccharides, polypeptides, polyvinylalcools, résines polyvinyliques, fluoropolymères, cires, les polyesters, polyéthers , et leurs mélanges.

Les filtres UV inorganiques utilisables dans la particule composite sont choisis parmi les oxydes métalliques.

De préférence, ces oxydes métalliques sont choisis parmi le dioxyde de titane $TiO_2$, l'oxyde de zinc ZnO, et l'oxyde de fer FeO. De manière particulièrement préférée, le filtre UV inorganique est le $TiO_2$.

Ces oxydes métalliques peuvent se présenter sous forme de particules, de taille moyenne généralement inférieure à 0,2 μm. Avantageusement, les particules de TiO2 utilisées présentent une taille moyenne inférieure ou égale

à 0,1 µm.

Ces oxydes métalliques peuvent aussi se présenter sous forme de couches, de préférence de multicouches d'épaisseur moyenne généralement inférieure à 0,2 µm.

De préférence, la teneur en particules composites de la composition selon l'invention va de 1 à 70 %, de préférence 1,5 à 45 %, de manière préférée de 2 à 20 % en poids par rapport au poids total de la composition cosmétique.

Les particules composites utilisables selon l'invention sont de préférence sphériques.

Elles peuvent être creuses, lisses, rugueuses, ou poreuses.

Lorsque les particules composites sont sous forme sphérique, elles se caractérisent par un diamètre moyen compris entre 0,1 µm et 30 µm, de préférence entre 0,2 µm et 20 µm et de manière encore préférée entre 0,3 µm et 10 µm, avantageusement entre 0,5 µm et 10 µm.

Par « sphérique », on entend que la particule présente un indice de sphéricité, c'est-à-dire le rapport entre son plus grand diamètre et son plus petit diamètre, inférieur à 1,2.

***Formes des particules composites***

Selon une première variante, les particules composites contiennent une matrice comprenant un matériau organique et/ou inorganique dans laquelle sont incluses des particules de filtre UV inorganique. De préférence, les particules composites sont constituées d'une matrice comprenant un matériau organique et /ou inorganique dans laquelle sont incluses des particules de filtre UV inorganique.

Selon cette réalisation, la matrice présente des inclusions et des particules de filtre UV inorganique sont placées dans les inclusions de la matrice.

De préférence, les particules composites sont sphériques et présentent des inclusions dans lesquelles sont placées des particules de filtre UV inorganique.

A titre de particules composites correspondant à cette variante, on peut citer les produits Sunsil TIN 50 et Sunsil TIN 40 commercialisés par la société SUNJIN CHEMICAL. Ces particules composites sphériques de taille moyenne comprise entre 2 et 7 µm sont formées de $TiO_2$ encapsulé dans une matrice de silice.

Selon une deuxième variante, les particules composites contiennent une matrice en un matériau organique et/ou inorganique recouverte d'au moins une couche de filtre UV inorganique pouvant être connectée à la matrice à l'aide d'un liant.

Selon cette deuxième variante, les particules de matrice sont, de manière préférée, de forme sphérique.

Selon cette deuxième variante, l'épaisseur moyenne de la couche de filtre UV inorganique est généralement d'une dizaine de nanomètres. L'épaisseur moyenne de la couche de filtre UV inorganique est avantageusement comprise entre $10^{-3}$ et 0,2 µm, de préférence entre $10^{-2}$ et 0,2 µm.

Selon une troisième variante, les particules composites contiennent un filtre UV inorganique recouvert d'au moins une couche d'un matériau organique et/ou inorganique. Selon cette troisième variante, les particules de filtre UV inorganique se caractérisent par une taille moyenne élémentaire généralement comprise entre $10^{-3}$ et 0,2 µm. Avantageusement, les particules d'oxydes métalliques utilisées présentent une taille moyenne élémentaire comprise entre $10^{-2}$ et 0,1 µm.

La matrice peut être formée d'un ou plusieurs matériaux organiques ou inorganiques. Il peut alors s'agir d'une phase continue de matériaux comme un alliage, c'est-à-dire une phase continue dans laquelle les matériaux ne sont plus dissociables, ou d'une phase discontinue de matériaux, par exemple constituée d'un matériau organique ou inorganique recouvert d'une couche d'un autre matériau organique ou inorganique différent.

Selon une variante, en particulier lorsque les particules composites comprennent une matrice recouverte d'une couche de filtre UV, les particules composites peuvent de plus être recouvertes d'un revêtement supplémentaire en particulier choisi parmi les matériaux biodégradables ou biocompatibles, les matériaux lipidiques comme par exemple les tensioactifs ou les émulsifiants, les polymères, et les oxydes.

Les particules composites utilisées selon l'invention sont de taille comprise entre 0,1 et 30 µm, de préférence entre 0,5 et 20 µm et de manière encore préférée entre 0,3 et 10 µm, avantageusement entre 0,5 et 10 µm.

De préférence, le filtre UV utilisé dans la particule composite est du $TiO_2$ ou un mélange de $TiO_2$ et de ZnO.

De préférence, la matrice de la particule composite sphérique contient un matériau ou un mélange de matériaux choisi parmi :

- la $SiO_2$,
- le polyméthacrylate de méthyle,
- les copolymères de styrène et d'un dérivé de (méth)acrylate d'alkyle en C1/C5,
- les polyamides tels que les nylons.

A titre de particules composites correspondant à cette variante, on peut citer les produits Sunsil TIN 50 et Sunsil

TIN 40 commercialisés par la société SUNJIN CHEMICAL. Ces particules composites sphériques de taille moyenne comprise entre 2 et 7 μm sont formées de TiO2 encapsulé dans une matrice de silice.

On peut encore citer les particules suivantes :

- Particules composites sphériques de taille moyenne comprise entre 4 et 8 μm, contenant du $TiO_2$ et du $SiO_2$ de nom commercial Eospoly TR commercialisé par la société CREATIONS COULEURS,
- Particules composites contenant du $TiO_2$ et une matrice de copolymère styrène/acrylate d'alkyle commercialisé sous le nom Eospoly UV TR22 HB 50 par la société CREATIONS COULEURS,
- Particules composites contenant du $TiO_2$ et du ZnO et une matrice de PMMA de nom commercial Sun PMMA-T50 commercialisé par la société SUNJIN CHEMICAL.

Parmi les particules composites utilisables selon l'invention, on peut encore citer les particules composites sphériques contenant du $TiO_2$ et du $SiO_2$ de nom commercial STM ACS-0050510, fourni par la société JGC Catalysts and Chemical.

De préférence, les filtres inorganiques sont choisis parmi le titanium dioxide, éventuellement enrobé d'aluminium hydroxide et d'acide stearique, le titanium dioxide éventuellement enrobé d'acide stéarique et d'alumine et leurs mélanges.

Selon un mode de réalisation particulier de l'invention, le au moins un filtre inorganique est présent dans la composition selon l'invention dans une teneur allant de 0,01 à 20 % en poids, de préférence de 0,1 à 10 % en poids, de manière préférée de 0,5 à 7 % en poids, et en particulier de 0,8 à 6 % en poids par rapport au poids total de la composition.

Les compositions conformes à la présente invention peuvent comprendre en outre des adjuvants cosmétiques classiques notamment choisis parmi les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, les charges, les polymères, les propulseurs, les agents alcalinisants ou acidifiants ou tout autre ingrédient habituellement utilisé dans le domaine cosmétique et/ou dermatologique.

Parmi les solvants organiques, on peut citer les alcools et polyols inférieurs. Ces derniers peuvent être choisis parmi les glycols comme le propylène glycol, le butylène glycol, le dipropylène glycol, la glycérine, le 1,3-propanediol, le penthylène glycol ou l'hexylène glycol.

Comme épaississants hydrophiles, on peut citer les polymères carboxyvinyliques tels que les Carbopols (Carbomers) et les Pemulen (Copolymère acrylate/C10-C30-alkylacrylate) ; les polyacrylamides comme par exemple les copolymères réticulés vendus sous les noms Sepigel 305 (nom C.T.F.A. : polyacrylamide/C13-C14 isoparaffin/Laureth 7) ou Simulgel 600 (nom C.T.F.A. : acrylamide / sodium acryloyldimethyltaurate copolymer / isohexadecane / polysorbate 80) par la société Seppic ; les polymères et copolymères d'acide 2-acrylamido 2-méthylpropane sulfonique, éventuellement réticulés et/ou neutralisés, comme le Poly(acide 2-acrylamido 2-méthylpropane sulfonique) commercialisé par la société Hoechst sous la dénomination commerciale « Hostacerin AMPS » (nom CTFA : ammonium polyacryloyldimethyl taurate ou le SIMULGEL 800 commercialisé par la société SEPPIC (nom CTFA : sodium polyacryolyldimethyl taurate / polysorbate 80 / sorbitan oleate) ; les copolymères d'acide 2-acrylamido 2-méthylpropane sulfonique et d'hydroxyethyl acrylate comme le SIMULGEL NS et le SEPINOV EMT 10 commercialisés par la société SEPPIC ; les dérivés cellulosiques tels que l'hydroxyéthylcellulose ; les polysaccharides et notamment les gommes telles que la gomme de Xanthane ; et leurs mélanges.

Comme épaississants lipophiles, on peut citer les polymères synthétiques tels que les poly C10-C30 alkyl acrylates vendu sous la dénomination « INTELIMER IPA 13-1 » et « INTELIMER IPA 13-6 » par la société Landec ou encore les argiles modifiées telles que l'hectorite et ses dérivés, comme les produits commercialisés sous les noms de Bentone.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions selon l'invention peuvent être préparées selon les techniques bien connues de l'homme de l'art. Elles peuvent se présenter en particulier sous forme d'émulsion directe, inverse ou multiple (H/E, E/H, H/E/H ou E/H/E). Elles peuvent éventuellement être conditionnées en aérosol et se présenter sous forme de spray.

De préférence, les compositions selon l'invention se présentent sous la forme d'une émulsion huile-dans-eau ou eau-dans huile et plus particulièrement des émulsions huile-dans-eau.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E). Les émulsions peuvent contenir également d'autres types de stabilisants comme par exemple des charges, des polymères gélifiants ou épaississants.

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les diméthicone copolyols tels que le mélange diméthicone et PEG/polypropylène glycol (PPG) 18/18 diméthicone, vendu sous la dénomination « X-22-6711D » par la société Shin Etsu, et les alkyl-dimethicone copolyols tels que le Laurylmethicone copolyol vendu sous la dénomination "Dow Corning 5200 Formulation Aid" par la société Dow Corning ; le Cetyl dimethicone copolyol tel que le produit vendu sous la dénomination Abil EM 90R par la société Goldschmidt et le mélange de cétyl diméthicone copolyol, d'isostéarate de polyglycérole (4 moles) et de laurate d'hexyle vendu sous la dénomination ABIL WE O9 par la société Goldschmidt. On peut y ajouter aussi un ou plusieurs co-émulsionnants, qui, de manière avantageuse, peuvent être choisis dans le groupe comprenant les esters alkylés de polyol ou les élastomères de silicone émulsionnant tels que le mélange de diméthicone et de polymère réticulé de diméthicone/ PEG-10/15 vendu sous la dénomination « KSG-210 ».

Comme esters alkylés de polyol, on peut citer notamment les esters de polyéthylèneglycol comme le PEG-30 Dipolyhydroxystearate tel que le produit commercialisé sous le nom Arlacel P135 par la socité ICI.

Comme esters de glycérol et/ou de sorbitan, on peut citer par exemple l'isostéarate de polyglycérol, tel que le produit commercialisé sous la dénomination Isolan GI 34 par la société Goldschmidt ; l'isostéarate de sorbitan, tel que le produit commercialisé sous la dénomination Arlacel 987 par la société ICI ; l'isostéarate de sorbitan et le glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) comme le mélange PEG-100 Stearate/ Glyceryl Stearate commercialisé par exemple par la société ICI sous la dénomination Arlacel 165 ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition auto-émulsionnante, comme décrit par exemple dans le document WO-A-92/06778.

Les exemples qui suivent illustent l'invention sans en limiter la portée.

Dans la description et les exemples, les pourcentages sont des pourcentages pondéraux. Les ingrédients sont mélangés, dans l'ordre et dans les conditions facilement déterminés par l'homme de l'art.

## Exemples

### Exemple 1 : Compositions conformes à l'invention

**Les compositions sont préparées en mélangeant les ingrédients suivants :**

| Nom INCI | Composition 1 (% en poids) | Composition 2 (% en poids) | Composition 3 (% en poids) | Composition 4 (% en poids) |
|---|---|---|---|---|
| Disodium EDTA | 0,1 | 0,1 | | 0,1 |
| Tocopherol | 0,5 | 0,5 | | 0,5 |
| Triethanoalmine | 0,33 | 0,85 | 0,07 | 0,59 |
| Conservateur | 1 | 1 | 1 | 1 |
| Octyldodecanol | 2 | | 2 | 2 |
| Cetearyl alcohol | 1 | 1 | 1 | 1 |
| Diisopropyl sebacate | | 3 | | 5 |
| Resveratrol | 0,25 | 0,25 | 0,25 | 0,25 |

(suite)

| Nom INCI | Composition 1 (% en poids) | Composition 2 (% en poids) | Composition 3 (% en poids) | Composition 4 (% en poids) |
|---|---|---|---|---|
| Ethylhexyl methoxycinnamate | | | 7,5 | |
| Butyl methoxydibenzoyl methane | 2,5 | 3 | | 3,5 |
| Ethylhexyl salicilate | | 5 | | 5 |
| Ethylhexyl triazone | 1 | 1 | | 1 |
| Terephtalylidene dicamphor sulfonic acid | 1,5 | 4.5 | 12 | 3 |
| Octocrylene | 7 | 2,5 | | 2,5 |
| Drometrizole trisiloxane | 1,5 | 0,5 | 1,5 | 0,5 |
| Bis-ethylhexyloxyphen ol methoxyphenyl triazine | | 1 | | 3 |
| Titanium dioxide (and) aluminium hydroxide (and) stearic acid | 1 | 2 | 1 | 5 |
| Xanthan gum | 0,2 | 0,2 | 0,2 | 0,2 |
| Ammonium polyacryloyldimeth yl taurate | 1,7 | 1,7 | 1,7 | 1,7 |
| Cyclohexasiloxane | 1 | | | 1 |
| Dimethicone (and) dimethiconol | 3 | 3 | 3 | 3 |
| Alcohol denat. | 5 | 5 | 5 | 5 |
| Glycerin | 4 | 4 | 4 | 4 |
| Propylene glycol | 8 | 8 | 8 | 8 |
| Stearic acid | 2 | 2 | 2 | 2 |
| Glyceryl stearate (and) PEG-100 stearate | 2 | 2 | 2 | 2 |
| PEG-20 stearate | 0,8 | 0,8 | 0,8 | 0,8 |
| Water | Qsp100 | Qsp100 | Qsp100 | Qsp100 |

Les émulsions des compositions 1 à 4 sont fines, homogènes, brillantes, lisses et stables dans le temps.

**Exemple 2 : Compositions non conformes à l'invention**

| Nom INCI | Composition 5 (% en poids) | Composition 6 (% en poids) | Composition 7 (% en poids) | Composition 8 (% en poids) |
|---|---|---|---|---|
| Disodium EDTA | 0,1 | 0,1 | 0,1 | 0,1 |
| Tocopherol | 0,5 | 0,5 | 0,5 | 0,5 |
| Triethanoalmine | 0,07 | 1,58 | 0,07 | 0,07 |
| Citric Acid | | 0,528 | | |
| Silica | 0,5 | 0,5 | 0,5 | 0,5 |

(suite)

| Nom INCI | Composition 5 (% en poids) | Composition 6 (% en poids) | Composition 7 (% en poids) | Composition 8 (% en poids) |
|---|---|---|---|---|
| Conservateur | 1 | 1 | 1 | 1 |
| Octyldodecanol | 2 | 2 | 2 | 2 |
| Cetearyl alcohol | 1 | 1 | 1 | 1 |
| Isopropyl lauroyl sarcosinate | 7.5 | | | |
| Resveratrol | 0,25 | 0,25 | 0,25 | 0,25 |
| Phenylbenzimidazole sulfonic acid | | 1,7 | | |
| Ethylhexyl salicilate | | | 5 | |
| Terephtalylidene dicamphor sulfonic | | 0,15 | | |
| acid | | | | |
| Octocrylene | | | 7 | |
| Bis-ethylhexyloxyphen ol methoxyphenyl triazine | | | | 1 |
| Butyl methoxydibenzoyl methane | | | 3 | |
| Ethylhexyl methoxycinnamate | | 7,5 | | |
| Titanium dioxide (and) stearic acid (and) alumina | | 1 | | |
| Xanthan gum | 0,2 | 0,2 | 0,2 | 0,2 |
| Ammonium polyacryloyldimeth yl taurate | 1,7 | 1,7 | 1,7 | 1,7 |
| Cyclohexasiloxane | 1 | 5 | 1 | 5 |
| Dimethicone (and) dimethiconol | 3 | 3 | 3 | 3 |
| Alcohol denat. | 5 | 5 | 5 | 5 |
| Glycerin | 4 | 4 | 4 | 4 |
| Stearic acid | 2 | 2 | 2 | 2 |
| Glyceryl stearate (and) PEG-100 stearate | 2 | 2 | 2 | 2 |
| PEG-20 stearate | 0,8 | 0,8 | 0,8 | 0,8 |
| Water | Qsp100 | Qsp100 | Qsp100 | Qsp100 |

**Exemple 3 : Caractéristiques des compositions 1 à 8**

| | Compositions conformes à l'invention | | | | Compositions non conformes à l'invention | | | |
|---|---|---|---|---|---|---|---|---|
| | 1 | 2 | 3 | 4 | 5 | 6 | 7 | 8 |
| Quantité de filtre UVA (% en poids) | 4 | 7,5 | 12 | 6,5 | 0 | 0,15 | 3 | 3 |
| Quantité de filtre UVB (% en poids) | 9,5 | 9 | 9 | 9 | 0 | 9,2 | 12 | 10 |

(suite)

| | Compositions conformes à l'invention | | | | Compositions non conformes à l'invention | | | |
|---|---|---|---|---|---|---|---|---|
| | 1 | 2 | 3 | 4 | 5 | 6 | 7 | 8 |
| SPF | 20 | 30 | 50 | 50 | / | 15 | 25 | 20 |
| PPD | 8 | 20 | 20 | 30 | / | 1 | 1 | 1 |
| Rapport SPF/PPD | 2,5 | 1,5 | 2,5 | 1,67 | / | 15 | 25 | 20 |
| % résiduel en *trans*-resvératrol (1) | 86 | 86 | 89 | 90 | <5 | 31 | 47 | 35 |
| (1)Le pourcentage résiduel en resveratrol est mesuré après une exposition de la composition à 5 J/cm$^2$ d'UV, ce qui correspond à une dose journalière. | | | | | | | | |

Les quatre compositions conformes à l'invention (compositions 1 à 4) présentent un pourcentage résiduel en resveratrol après une exposition de la composition à 5 J/cm$^2$ d'UV, très élevé ce qui signifie que ce dernier se dégrade très peu au contact de la lumière du jour.

Lorsque la composition ne comprend pas de filtre UV (composition 5), la dégradation du resveratrol après une exposition de la composition à 5 J/cm$^2$ d'UV, est quasi totale ce qui signifie que ce dernier se dégrade beaucoup au contact de la lumière du jour.

Lorsque la composition possède un facteur de protection solaire UVA (PPD) faible (compositions 6, 7 et 8), on observe également une dégradation importante du *trans*-resvératrol.

**Exemple 4 : Composition conforme à l'invention exempte de Ti02**

| Nom INCI | % en poids |
|---|---|
| Dimyristyl tartrate (and) cetearyl alcohol (and) $C_{12-15}$ pareth-7 (and) PPG-25-laureth-25 | 2,00 |
| Caprylic/capric trigyceride | 2,00 |
| Drometrizole trisiloxane | 1,50 |
| Ethylhexyl triazone | 1,00 |
| Butyl methoxydibenzoyl methane | 2,50 |
| Octocrylene | 7,00 |
| Prunus armeniaca kernel oil | 1,00 |
| Glyceryl stearate (and) PEG-100 stearate | 0,60 |
| Cyclohexasiloxane | 5,00 |
| Tocopherol | 0,50 |
| Propanediol | 3,00 |
| Disodium EDTA | 0,20 |
| Phenoxyethanol | 0,50 |
| Potassium cetyl phosphate | 1,00 |
| Terephtalylidene dicamphor sulfonic acid | 1,50 |
| Triethanolamine | 0,26 |
| Water | Qsp 100 |
| Acrylates/$C_{10-30}$ alkyl acrylate crosspolymer | 0,43 |
| Triethanolamine | 0,83 |

(suite)

| Nom INCI | % en poids |
|---|---|
| Polyacrylamide (and) $C_{13-14}$ isoparaffin (and) laureth-7 | 1,00 |
| Hydroxyethyl acrylate/sodium acryloyldimethyl taurate copolymer | 0,50 |
| Alcohol not denat. | 5,00 |
| Lactic acid | 0,53 |
| Resveratrol | 1,00 |

Cette composition est homogène et stable dans le temps.

## Revendications

1. Composition cosmétique ou dermatologique comprenant, dans un milieu physiologiquement acceptable, au moins du *trans*-resvératrol et/ou au moins un dérivé de *trans*-resvératrol choisi parmi les esters, les glucosides et les phosphates du *trans*-resvératrol, et un système filtrant organique comprenant au moins un filtre UVA et au moins un filtre UVB et/ou au moins un filtre mixte UVA-UVB,
ladite composition possédant un SPF supérieur ou égal à 15 et un PPD supérieur ou égal à 5,
ledit filtre UVA étant un filtre hydrosoluble UVA.

2. Composition selon la revendication 1, dans laquelle ledit *trans*-resvératrol et/ou dérivé(s) de *trans*-resvératrol est présent dans une teneur allant de 0,001 à 10 %, de préférence de 0,01 à 5 % en poids, de manière préférée de 0,05 à 2 % en poids par rapport au poids total de la composition.

3. Composition selon la revendication 1 ou 2, **caractérisée en ce que** le rapport SPF/PPD va de 1 à 3, de préférence, de 1,3 à 2,7 et mieux de 1,5 à 2,5.

4. Composition selon l'une quelconque des revendications précédentes, **caractérisée en ce que** ledit au moins un filtre UVA est choisi parmi les dérivés hydrosolubles du camphre, de préférence l'acide téréphtalylidène dicamphre sulfonique

5. Composition selon l'une quelconque des revendications précédentes, **caractérisée en ce qu'**elle comprend en outre au moins un filtre UVA choisi parmi les dérivés hydrophobes du dibenzoylméthane, de préférence le butyl méthoxydibenzoylméthane.

6. Composition selon l'une quelconque des revendications précédentes, **caractérisée en ce que** ledit au moins un filtre UVA est présent dans une teneur allant de 1 à 20 % et en particulier de 2 à 15 % en poids par rapport au poids total de la composition.

7. Composition selon la revendication 1 à 3, **caractérisée en ce que** ledit au moins un filtre UVB est un filtre hydrophobe UVB, de préférence choisi parmi les dérivés salicyliques, les cinnamates, les dérivés de β,β'-diphénylacrylate, les dérivés de triazine et leurs mélanges.

8. Composition selon la revendication 1, 2, 3, ou 7, **caractérisée en ce que** ledit au moins un filtre UVB est choisi parmi l'éthylhexyl salicylate, l'éthylhexyl méthoxycinnamate, l'octocrylène, l'éthylhexyl triazone et leurs mélanges.

9. Composition selon l'une quelconque des revendications précédentes 1 à 3 ou 7 ou 8, **caractérisée en ce que** ledit au moins un filtre UVB est présent dans une teneur allant de 1 à 20 %, en particulier de 5 à 15 % et de préférence, de 7 à 10 % en poids par rapport au poids total de la composition.

10. Composition selon l'une quelconque des revendications précédentes, **caractérisée en ce que** ledit système filtrant organique comprend au moins un filtre mixte UVA-UVB, de préférence choisi parmi les filtres mixtes UVA-UVB hydrophobes, avantageusement le filtre mixte UVA-UVB hydrophobe est choisi parmi les dérivés du phényl benzotriazole et les dérivés bis-résorcinyl triazines, de manière préférée parmi le drometrizole trisiloxane, le bis-ethylhexyloxyphénol méthoxyphenyl triazine, et leurs mélanges.

11. Composition selon l'une quelconque des revendications précédentes, **caractérisée en ce que** ledit système filtrant organique comprend au moins un filtre choisi parmi l'acide téréphtalylidène dicamphre sulfonique, le drometrizole trisiloxane et leurs mélanges.

12. Composition selon l'une quelconque des revendications précédentes, **caractérisée en ce qu'**elle se présente sous la forme d'une émulsion huile-dans-eau ou eau-dans huile et plus particulièrement des émulsions huile-dans-eau.

13. Composition selon l'une quelconque des revendications précédentes, **caractérisée en ce que** ledit système filtrant organique comprend au moins un filtre hydrophobe, de préférence au moins un filtre hydrophobe UVB.

14. Procédé de stabilisation, en particulier de photostabilisation, d'une composition comprenant du *trans*-resvératrol et/ou au moins un dérivé de *trans*-resvératrol choisi parmi les esters, les glucosides et les phosphates du *trans*-resvératrol comprenant une étape lors de laquelle on ajoute à ladite composition un système filtrant organique comprenant au moins un filtre UVA et au moins un filtre UVB et/ou au moins un filtre mixte UVA-UVB, ledit filtre UVA étant un filtre hydrosoluble UVA, en une quantité telle que le SPF de la composition est porté à une valeur supérieure ou égale à 15 et le PPD de la composition est porté à une valeur supérieure ou égale 5.

15. Utilisation d'un système filtrant comprenant au moins un filtre UVA et au moins un filtre UVB et/ou au moins un filtre mixte UVA-UVB, ledit filtre UVA étant un filtre hydrosoluble UVA, pour stabiliser notamment photostabiliser une composition comprenant du *trans*-resvératrol et/ou au moins un dérivé de *trans*-resvératrol choisi parmi les esters, les glucosides et les phosphates du *trans*-resvératrol.

## Patentansprüche

1. Kosmetische oder dermatologische Zusammensetzung, umfassend, in einem physiologisch verträglichen Medium, mindestens ein *trans*-Resveratrol und/oder mindestens ein *trans*-Resveratrol-Derivat, ausgewählt aus *trans*-5-Resveratrolestern, -glucosiden und -phosphaten, und ein organisches Filtersystem, umfassend mindestens einen UVA-Filter und mindestens einen UVB-Filter und/oder mindestens einen UVA-UVB-Mischfilter,
wobei die Zusammensetzung einen SPF größer oder gleich 15 und eine PPD größer oder gleich 5 aufweist,
wobei der UVA-Filter ein wasserlöslicher UVA-Filter ist.

2. Zusammensetzung nach Anspruch 1, wobei das *trans*-Resveratrol und/oder das/die *trans*-Resveratrol-Derivat(e) in einem Gehalt im Bereich von 0,001 bis 10 %, vorzugsweise von 0,01 bis 5 Gew.-%, vorzugsweise von 0,05 bis 2 Gew.-%, bezogen auf das Gesamtgewicht der Zusammensetzung, vorhanden ist.

3. Zusammensetzung nach Anspruch 1 oder 2, **dadurch gekennzeichnet, dass** das SPF/PPD-Verhältnis von 1 bis 3, vorzugsweise von 1,3 bis 2,7 und besser von 1,5 bis 2,5 beträgt.

4. Zusammensetzung nach einem der vorangehenden Ansprüche,
**dadurch gekennzeichnet, dass** der mindestens eine UVA-Filter ausgewählt ist aus wasserlöslichen Kampferderivaten, vorzugsweise Terephthalylidendikampfersulfonsäure.

5. Zusammensetzung nach einem der vorangehenden Ansprüche, **dadurch gekennzeichnet, dass** sie ferner mindestens einen UVA-Filter umfasst, ausgewählt aus hydrophoben Dibenzoylmethanderivaten, vorzugsweise Butylmethoxydibenzoylmethan.

6. Zusammensetzung nach einem der vorangehenden Ansprüche, **dadurch gekennzeichnet, dass** der mindestens eine UVA-Filter in einem Gehalt im Bereich von 1 % bis 20 % und insbesondere von 2 bis 15 Gew.-%, bezogen auf das Gesamtgewicht der Zusammensetzung, vorhanden ist.

7. Zusammensetzung nach Anspruch 1 bis 3, **dadurch gekennzeichnet, dass** der mindestens eine UVB-Filter ein hydrophober UVB-Filter ist, vorzugsweise ausgewählt aus Salicylderivaten, Cinnamaten, ß,ß'-Diphenylacrylatderivaten, Triazinderivaten und Mischungen davon.

8. Zusammensetzung nach Anspruch 1, 2, 3 oder 7, **dadurch gekennzeichnet, dass** der mindestens eine UVB-Filter

ausgewählt ist aus Ethylhexylsalicylat, Etbylhexylmethoxycinnamat, Octocrylen, Ethylhexyltriazon und Mischungen davon.

**9.** Zusammensetzung nach einem der vorangehenden Ansprüche 1 bis 3 oder 7 oder 8, **dadurch gekennzeichnet, dass** der mindestens eine UVB-Filter in einem Gehalt von 1 bis 20%, insbesondere von 5 bis 15% und vorzugsweise von 7 bis 10%, bezogen auf das Gesamtgewicht der Zusammensetzung, vorhanden ist.

**10.** Zusammensetzung nach einem der vorangehenden Ansprüche, **dadurch gekennzeichnet, dass** das organische Filtersystem mindestens einen UVA-UVB-Mischfilter umfasst, der vorzugsweise ausgewählt ist aus hydrophoben UVA-UVB-Mischfiltern, wobei der hydrophobe UVA-UVB-Mischfilter vorteilhafterweise aus Phenylbenzotriazolderivaten und Bis-Resorcinyltriazin-Derivaten, vorzugsweise aus Drometrizol-Trisiloxan, Bis-Ethylhexyloxyphenol-Methoxyphenyltriazin und Mischungen davon.

**11.** Zusammensetzung nach einem der vorangehenden Ansprüche, **dadurch gekennzeichnet, dass** das organische Filtersystem mindestens einen Filter umfasst, ausgewählt aus Terephthalylidendikampfersulfonsäure, Drometrizol-Trisiloxan und Mischungen davon.

**12.** Zusammensetzung nach einem der vorangehenden Ansprüche, **dadurch gekennzeichnet, dass** sie in Form einer Öl-in-Wasser- oder Wasser-in-Öl-Emulsion und insbesondere einer Öl-in-Wasser-Emulsion vorliegt.

**13.** Zusammensetzung nach einem der vorangehenden Ansprüche, **dadurch gekennzeichnet, dass** das organische Filtersystem mindestens einen hydrophoben Filter, vorzugsweise mindestens einen hydrophoben UVB-Filter, umfasst.

**14.** Verfahren zur Stabilisierung, insbesondere zur Photostabilisierung, einer Zusammensetzung, die *trans*-Resveratrol und/oder mindestens ein *trans*-Resveratrol-Derivat umfasst, ausgewählt aus Estern, Glucosiden und Phosphaten von *trans*-Resveratrol, umfassend einen Schritt, wobei ein organisches Filtersystem, das mindestens einen UVA-Filter und mindestens einen UVB-Filter und/oder mindestens einen UVA-UVB-Mischfilter umfasst, zu der Zusammensetzung hinzugefügt wird, wobei der UVA-Filter ein wasserlöslicher UVA-Filter in einer solchen Menge ist, dass der SPF der Zusammensetzung auf einen Wert größer als oder gleich 15 und der PPD der Zusammensetzung auf einen Wert größer als oder gleich 5 erhöht wird.

**15.** Verwendung eines Filtersystems, das mindestens einen UVA-Filter und mindestens einen UVB-Filter und/oder mindestens einen UVA-UVB-Mischfilter umfasst, wobei der UVA-Filter ein wasserlöslicher UVA-Filter ist, um insbesondere eine Zusammensetzung zu stabilisieren, die *trans*-Resveratrol und/oder mindestens ein Derivat von *trans*-Resveratrol, ausgewählt aus den Estern, Glucosiden und Phosphaten von *trans*-Resveratrol, umfasst.

## Claims

**1.** Cosmetic or dermatological composition comprising, in a physiologically acceptable medium, at least *trans*-resveratrol and/or at least one *trans*-resveratrol derivative selected from among the esters, glucosides and phosphates of *trans*-resveratrol, and an organic filtering system comprising at least one UVA filter and at least one UVB filter and/or at least one mixed UVA-UVB filter,
said composition having a SPF of 15 or higher and PPD of 5 or higher,
said UVA filter being a water-soluble UVA filter.

**2.** The composition according to claim 1, wherein said *trans*-resveratrol and/or *trans*-resveratrol derivative(s) is contained in a content ranging from 0.001 to 10 %, preferably from 0.01 to 5% by weight, more preferably from 0.05 to 2 % by weight relative to the total weight of the composition.

**3.** The composition according to claim 1 or 2, **characterized in that** the SPF/PPD ratio ranges from 1 to 3, preferably from 1.3 to 2.7 and better still from 1.5 to 2.5.

**4.** The composition according to any of the preceding claims, **characterized in that** said at least one UVA filter is selected from among water-soluble derivatives of camphor, preferably terephthalylidene dicamphor sulfonic acid.

**5.** The composition according to any of the preceding claims, **characterized in that** it further comprises at least one

UVA filter selected from among hydrophobic derivatives of dibenzoylmethane, preferably butyl methoxydibenzoylmethane.

**6.** The composition according to any of the preceding claims, **characterized in that** said at least one UVA filter is contained in a content ranging from 1 to 20 %, and in particular from 2 to 15 % by weight relative to the total weight of the composition.

**7.** The composition according to claim 1 to 3, **characterized in that** said at least one UVB filter is a hydrophobic UVB filter, preferably selected from among salicylic derivatives, cinnamates, derivatives of β, β'-diphenylacrylate, derivatives of triazine and mixtures thereof.

**8.** The composition according to claim 1, 2, 3 or 7, **characterized in that** said at least one UVB filter is selected from among ethylhexyl salicylate, ethylhexyl methoxycinnamate, octocrylene, ethylhexyl triazone and mixtures thereof.

**9.** The composition according to any of the preceding claims 1 to 3 or 7 or 8, **characterized in that** said at least one UVB filter is contained in a content ranging from 1 to 20 %, in particular from 5 to 15 % and preferably from 7 to 10 % by weight relative to the total weight of the composition.

**10.** The composition according to any of the preceding claims, **characterized in that** said organic filtering system comprises at least one mixed UVA-UVB filter, preferably selected from among hydrophobic mixed UVA-UVB filters, advantageously the hydrophobic mixed UVA-UVB filter is selected from among derivatives of phenyl benzotriazole and derivatives of bis-resorcinyl triazine, preferably from among drometrizole trisiloxane, bis-ethylhexyloxyphenol methoxyphenyl triazine and mixtures thereof.

**11.** The composition according to any of the preceding claims, **characterized in that** said organic filtering system comprises at least one filter selected from among terephthalylidene dicamphor sulfonic acid, drometrizole trisiloxane and mixtures thereof.

**12.** The composition according to any of the preceding claims, **characterized in that** it is in the form of an oil-in-water or water-in-oil emulsion, and more particularly oil-in-water emulsions.

**13.** The composition according to any of the preceding claims, **characterized in that** said organic filtering system comprises at least one hydrophobic filter, preferably at least one UVB hydrophobic filter.

**14.** Method for stabilising, in particular photo-stabilising, a composition comprising *trans*-resveratrol and/or at least one *trans*-resveratrol derivative selected from among the esters, glucosides and phosphates of *trans*-resveratrol, comprising a step at which the addition is made to said composition of an organic filtering system comprising at least one UVA filter and at least one UVB filter and/or at least one mixed UVA-UVB filter, said UVA filter being a water-soluble UVA filter, in an amount such that the SPF of the composition is brought to a value of 15 or higher and the PPD of the composition is brought to a value of 5 or higher.

**15.** Use of a filtering system comprising at least one UVA filter and at least one UVB filter and/or at least one mixed UVA-UVB filter, said UVA filter being a water-soluble UVA filter, to stabilise and particularly photo-stabilise a composition comprising *trans*-resveratrol and/or at least one *trans*-resveratrol derivative selected from among the esters, glucosides and phosphates of *trans*-resveratrol.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 669323 A
- US 2463264 A
- US 6225467 B
- WO 2004085412 A
- WO 06035000 A
- WO 06034982 A
- WO 06034991 A
- WO 06035007 A
- WO 2006034992 A
- WO 2006034985 A
- WO 9206778 A


### Littérature non-brevet citée dans la description


- Symetrical Triazine Derivatives. IP.COM Journal. IP.COM INC WEST HENRIETTA, 20 Septembre 2004
- *Cosmetics & Toiletries,* Février 1990, vol. 105, 53-64